(19) Europäisches Patentamt
European Patent Office
Office européen des brevets

(11) Veröffentlichungsnummer: **0 123 233**
**B1**

(12) # EUROPÄISCHE PATENTSCHRIFT

(45) Veröffentlichungstag der Patentschrift: **02.11.88**

(21) Anmeldenummer: **84104205.4**

(22) Anmeldetag: **13.04.84**

(51) Int. Cl.[4]: **C 07 C 37/06,** C 07 C 37/07, C 07 C 39/07, C 07 C 39/15, C 07 C 39/06

(54) **Verfahren zur Herstellung von substituierten Phenolen.**

(30) Priorität: **20.04.83 DE 3314372**

(43) Veröffentlichungstag der Anmeldung: **31.10.84 Patentblatt 84/44**

(45) Bekanntmachung des Hinweises auf die Patenterteilung: **02.11.88 Patentblatt 88/44**

(84) Benannte Vertragsstaaten: **CH DE FR GB LI NL**

(56) Entgegenhaltungen:
**DE-A-2 045 882**
**DE-A-2 064 097**
**FR-A-1 509 921**
**GB-A-1 258 963**
**US-A-3 935 282**
**US-A-4 088 702**

**CHEMICAL ABSTRACTS, Band 84, Nr. 5, 2. Februar 1976, Seite 418, Zusammenfassung Nr. 30575t, Columbus, Ohio, US; N.S. KOZLOV et al.: "Dehydrogenation of the cyclohexanol-cyclohexanone mixture to phenol" & ZH. PRIKL. KHIM. (LENINGRAD) 1975, 48(9), 2097-9**

(73) Patentinhaber: **BASF Aktiengesellschaft, Carl-Bosch- Strasse 38, D-6700 Ludwigshafen (DE)**

(72) Erfinder: **Goetz, Norbert, Dr., Schoefferstrasse 25, D-6520 Worms 1 (DE)**
Erfinder: **Laas, Harald, Dr., Woehlerstrasse 14 a, D-6701 Maxdorf (DE)**
Erfinder: **Tavs, Peter, Dr., Neuhofener Strasse 45, D-6703 Limbergerhof (DE)**
Erfinder: **Hupfer, Leopold, Dr., Waltershoehe 3, D-6701 Freidelsheim (DE)**
Erfinder: **Baer, Karl, Dr., Kastanienweg 1, D-6940 Weinheim (DE)**

**EP 0 123 233 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die vorliegende Erfindung betrifft ein verbessert es Verfahren zur Herstellung von substituierten Phenolen durch Dehydrierung sechsgliedriger cyclischer Alkohole, gesättigter oder ungesättigter Ketone an Palladium- oder Platin-Trägerkatalysatoren.

Die Aromatisierung sechsgliedriger cyclischer Verbindungen zu Phenolen über Abspaltungsreaktionen, wie beispielsweise Dehydrierungen, Dehydratisierungen oder Dehydrohalogenierungen stellt eine seit langem bekannte Methode dar (Houben-Weyl, "Methoden der organischen Chemie" Band 6/1 c, Teil 2, Seiten 670 ff). Die technisch interessanteste Variante dieser Abspaltungsreaktionen ist zweifellos die Dehydrierung. In der Literatur ist die Dehydrierung einer großen Anzahl unterschiedlich substituierter Cyclohexanole, Cyclohexanone oder Cyclohexenone beschrieben (loc. cit., Seiten 675 - 682), wobei sicherlich unter anderem der Üerführung von 2, 5, 6 - oder 2, 3, 6-Trimethylcyclohexenon in das 2, 3, 6-Trimethylphenol - einem Vorprodukt für Vitamin E - eine größere Bedeutung beizumessen ist.

Ein recht vorteilhaftes Verfahren für die Herstellung dieses 2, 3, 6-Trimethyl-phenol wird in der britischen Patentschrift 1 258 963 beschrieben.

Gemäß diesem Verfahren wird das Trimethylphenol dadurch erhalten, daß man Diethylketon in Gegenwart von basischen Mitteln mit Methylvinylketon oder Crotonaldehyd umsetzt und das erhaltene 2, 3, 6 - bzw. 2, 3, 6-Trimethyl-2-cyclohexen-1-on in der Gasphase dehydriert.

Als Katalysatoren für die Gasphasendehydrierung werden in diesem Patent Metalle der VIII. Gruppe des Periodensystems wie Eisen, Cobalt, Nickel, Rhodium, Platin, Palladium oder Metalle und Metalloxide der 1. Nebengruppe des Periodensystems, wie Kupfer oder Silber, genannt. Weiterhin heißt es in der genannten Patentschrift, daß die metallischen Dehydrierungskatalysatoren zweckmäßig auf einem üblichen Tragermaterial wie Aluminiumoxid, Kieselsäuregel oder Aktivkohle angewendet werden. Gemäß den Beispielen werden Palladiumkatalysatoren mit Kieselgel oder Aktivkohle als Trägermaterial angewendet und recht gute Ausbeuten an 2, 3, 6-Trimethylphenol erzielt. Führt man die genannte Dehydrierung mit den hier beschriebenen Katalysatoren in einer kontinuierlich arbeitenden Apparatur durch, so stellt man fest, daß man mit diesen Katalysatoren nur kurzzeitig optimale Ergebnisse erzielen kann, da die Katalysatoraktivität schon nach wenigen Stunden merklich abnimmt.

Ganz allgemein kann man sagen, daß die Dehydrierung von cyclischen Alkoholen oder Ketonen offensichtlich um so leichter verläuft, je stärker der aromatische Ring vorgebildet ist. Nach Literangaben werden für diese Umsetzung in der Mehrzahl Edelmetall-Trägerkatalysatoren, insbesondere Palladium auf Kohleträgern, verwendet. Der Grund für die starke Bevorzugung der neutralen Kohleträger dürfte darin zu suchen sein, daß saure Träger, wie beispielsweise Kieselgel, Dehydratisierungen als Nebenreaktion und alkalische Träger, wie beispielsweise Magnesiumoxid oder Calciumoxid, bei Umsetzungen mit Ketonen unerwünschte Kondensationsreaktionen begünstigen können.

Es war daher die Aufgabe der Erfindung, einen Dehydrierkatalysator zu entwickeln, an dem die Dehydrierung sechsgliedriger cyclischer Alkohole und Ketone auch in einem kontinuierlich arbeitenden Dehydrierungsprozeß vorteilhaft ausgeführt werden kann, d.h. einen formstabilen Katalysator mit dem die gewünschte Dehydrierung in hoher Ausbeute und Selektivität gelingt und der sich zusätzlich durch eine möglichst lange Katalysatorstandzeit ohne nennenswerten Aktivitätsabfall auszeichnet.

Es wurde nun überraschenderweise gefunden, daß die Dehydrierung sechsgliedriger cyclischer Alkohole und gesättigter oder ungesättigter Ketone mit ausgezeichneten Umsätzen und Ausbeuten gelingt, wenn man einen Palladium- oder Platinkatalysator auf einem Aluminiumspinell als Träger verwendet. Ein solcher Katalysator zeichnet sich insbesondere durch gute Formstabilität und Langanhaltende Aktivität aus.

Gegenstand der Erfindung ist dementsprechend ein verbessertes Verfahren zur Herstellung von substituierten Phenolen der allgemeinen Formel I

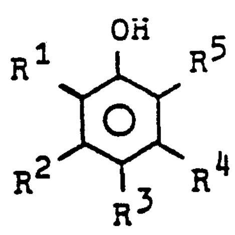 I,

in der $R^1$ bis $R^5$ Wasserstoff oder einen aliphatischen oder aromatischen Kohlenwasserstoffrest bedeuten, durch Dehydrieren eines substituierten cyclischen Alkohols der allgemeinen Formel II

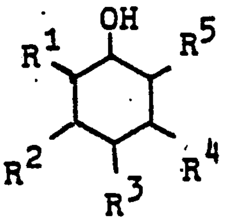 II,

oder eines subsistuierten cyclischen Ketons der allgemeinen Formel III,

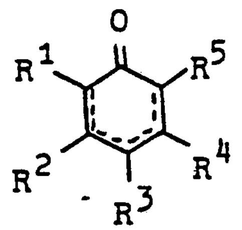 III,

in der eine der gestrichelten Linien für eine zusätzliche C-C-Bindung stehen kann, in der Gasphase bei Temperaturen von 150 bis 380° C an einem Edelmetall-Trägerkatalysator, das dadurch gekennzeichnet ist, daß man als Dehydrierungskatalysator einen Palladium- oder Platinkatalysator auf einem Aluminiumspinell als Katalysatorträger verwendet.

Besonders vorteilhaft gelingt das erfindungsgemäße Verfahren, wenn man einen Palladium- oder Platinkatalysator auf einem Aluminiumspinell als Katalysatorträger verwendet, der eine innere Oberfläche nach BET von 5 bis 150 $m^2/g$ aufweist. Die Dehyrierung von ungesättigten cyclischen Ketonen, d.h. Cyclohexenonen der allgemeinen Formel III gelingt besonders vorteilhaft, wenn man einen Palladium- oder Platinkatalysator auf einem Aluminiumspinell als Katalysatorträger verwendet, der eine innere Oberfläche nach BET von 10 bis 50 $m^2/g$, aufweist. Besonders geeignet als Katalysatorträger sind Aluminiumspinelle, die außer Aluminium als weiteres spinellbildendes Metall noch Magnesium, Zink, Cobalt oder Nickel enthalten.

Bezüglich von Methoden zur Bestimmung der inneren Oberfläche nach BET verweisen wir auf die Originalliteratur: Brunauer, Emmett und Teller im J.Amer.Chem.Soc. 60 (1938), S. 309.

Nach dem erfindungsgemäßen Verfahren werden substituierte Phenole bei vollständigem Umsatz frei von Nebenprodukten, die durch Dehydratisierungs- oder Kondensationsreaktionen entstehen können, gewonnen. Vorteilhaft ist weiterhin die lange Lebensdauer der verwendeten Palladium- und Platinkatalysatoren auf Aluminiumspinellträgern; so ist beispielsweise nach einer Dauerbeanspruchung von über 1500 Betriebsstunden kein Aktivitätsabfall zu verzeichnen. Palladiumkatalysatoren auf Aluminiumspinellträgern sind bereits bekannt; sie wurden bisher für andere Zwecke, z. B. für die selektive Gasphasenhydrierung von Phenol zu Cyclohexanon (vgl. DE-OS-2 045 882) oder für die Herstellung von Brenzcatechin oder Brenzcatechinmonoethern aus Cyclohexanonderivaten (DE-OS-2 064 097, DE-OS-2 126 150) verwendet. Für die zuletzt genannte Umsetzung liefert allerdings nur das Benutzen von ganz speziellen mit Chrom oder Titan dotierten Palladiumkatalysatoren auf Lithiumaluminiumspinell-Trägern befriedigende Ausbeuten.

Als Ausgangsverbindungen der allgemeinen Formeln II bzw. III kommen im wesentlichen solche in Betracht, in denen $R^1$ bis $R^5$ Wasserstoff, einen aliphatischen Kohlenwasserstoffrest mit 1 bis 10, vorzugsweise 1 bis 4 C-Atomen, insbesondere eine Methylgruppe bedeuten, oder für einen aromatischen Kohlenwasserstoffrest mit 6 bis 15 C-Atomen, vorzugsweise eine gegebenenfalls durch eine oder mehrere niedere Alkylgruppen substituierte Phenylgruppe stehen. Von besonderem Interesse sind Verbindungen der Formeln II und III, in denen jeweils 1 bis 3 der Reste $R^1$ bis $R^5$ einen Kohlenwasserstoffrest, die anderen Wasserstoff bedeuten.

Beispielsweise seien folgende geeignete Ausgangsverbindungen genannt. 2-, 3- und 4-Methyl-cyclohexanol, 2,6-Dimethyl-cyclohexanol, 3,5-Dimethyl-cyclohexanol, 3,4-Dimetyl-cyclohexanol, 2,3,6-Trimethyl-cyclohexanol, 2,5,6-Trimethyl-cyclohexanol, 2,6-Diisopropyl-cyclohexanol, 2-Methyl-6-ethyl-cyclohexanol, 2-Methyl-6-butyl-cyclohexanol, 2,6-Dimethyl-3-phenyl-cyclohexanol, 2-, 3- und 4-Metyl-cyclohexanon, 2-Methyl-cyclohex-2-en-1-on, 3-Methyl-cyclohex-2-en-1-on, 4-Methyl-cyclohex-2-en-1-on, 2,6-Dimethyl-cyclohexanon, 2,6-Dimethyl-cyclohex-2-en-1-on, 3,5-Dimethyl-cyclohexanon, 3,4-Dimethyl-cyclohexanon, 2,3,6-Trimethyl-cyclohexanon, 2,3,6-Trimethyl-cyclohex-2-en-1-on, 2,5,6-Trimethyl-cyclohexanon, 2,5,6-Trimethyl-cyclohex-2-en-1-on, 2,6-Diisopropyl-cyclohexanon, 2-Methyl-6-ethyl-cyclohexanon, 2-Methyl-6-ethyl-cyclohex-2-en-1-on, 2-Methyl-6-butylcyclohexanon, 2,6-Dimethyl-3-phenyl-cyclohex-5-en-1-on.

Ein großer Vorteil des erfindungsgemäßen Verfahrens besteht darin, daß auch ohne Schwierigkeiten Mischungen aus cyclischen Alkoholen und cyclischen Ketonen mit entsprechendem Substitutionsmuster eingesetzt werden können (siehe Beispiel 3).

Das nach dem Verfahren der Erfindung verwendete Katalysatorsystem enthält Palladium oder Platin auf einem Aluminiumspinellträger. Der Edelmetallgehalt des Katalysators, bezogen auf das Trägermaterial, liegt gewöhnlich zwischen 0,05 und 15, vorzugsweise 0,1 bis 2 Gew. %.

Aluminiumspinelle erhält man allgemein durch Umsetzung von Aluminiumoxid mit Verbindungen von ein- oder zweiwertigen Metallen, wie Lithium, Magnesium, Cobalt, Nickel, Mangan oder Zink (s. Gmelin, System-Nr. 35, Al, Tl, 1934 - 1935 und Ullmanns Encyklopädie der technischen Chemie, 3. Auflage (1955), Band 6, Seiten 242 - 244). Prinzipiell ergeben sich verschiedene Herstellmöglichkeiten für den Katalysator.

a) Auf strang- oder kugelförmiges γ-Aluminiumoxid wird die zweite spinellbildende Komponente in der gewünschten Menge durch Tränken mit entsprechenden wäßrigen Nitrat- oder Formiatlösungen aufgebracht und das erhaltene Produkt bei 150°C getrocknet. Um Spinellbildung zu erreichen, muß dieses Produkt nun mehrere Stunden (2 bis 10 Stunden) auf Temperaturen von 900 bis 1300°C, vorzugsweise 1000 bis 1250°C, erhitzt werden.

b) Die beiden Komponenten werden in Form ihrer oxide, z. B. γ-Aluminiumoxid und Magnesiumoxid, in dem benötigten Mengenverhältnis miteinander verknetet und dann bei Temperaturen zwischen 900 und 1300°C, vorzugsweise 1000 und 1250°C, getempert.

c) Die beiden Komponenten werden aus wäßrigen Salzlösungen gemeinsam als Hydroxide ausgefällt und anschließend - zur Spinellbildung - bei Temperaturen zwischen 900 und 1300°C, vorzugsweise 1000 und 1250°C erhitzt.

In Abhängigkeit von Temperaturen und Temperzeit werden nach den Herstellungsmethoden a) bis c) Katalysatorträger erhalten, die innere Oberflächen zwischen 5 und 150 $m^2/g$ besitzen. Speziell zur Umsetzung von substituierten Cyclohexenonen zu substituierten Phenolen nach dem erfindungsgemäßen Verfahren sollten Träger mit inneren Oberflächen von 10 bis 50 $m^2/g$ verwendet werden, da nur unter dieser Voraussetzung die bereits erwähnten langen Katalysator- Standzeiten erreicht werden (Herstellung dieses Katalysators: siehe Beispiel 1). Für die Umsetzung der substituierten Cyclohexanone und für die Dehydrierung der substituierten Cyclohexanole ist diese Einschränkung im verwendeten Trägermaterial nicht erforderlich.

Das Aufbringen des Edelmetalls auf den fertigen Träger erfolgt vorteilhaft so, daß man das Trägermaterial mit einer wäßrigen Edelmetallnitratlösung tränkt und dann - nach Aufnahme der gesamten Lösungsmengebei 100 bis 140°C, vorzugsweise bei 110 bis 120°C trocknet. Die Katalysatoren werden in Form von Kugeln oder Strängen mit einem Durchmesser von beispielsweise 3 mm und einer Länge von 10 mm oder aber als Pulver (für Wirbelöfen) - je nach vorgesehenem Anwendungszweck - eingesetzt. Die Aktivierung des Katalysators erfolgt durch mehrstündiges Erhitzen in Gegenwart von Wasserstoff auf die Reaktionstemperatur.

Zur Durchführung der erfindungsgemäßen Umsetzung werden die Ausgangsgangsproduktes am Reaktoreingang noch ein Fremdgas, wie Wasserstoff- oder Stickstoff, vorzugsweise Wasserstoff zuzudosieren, wobei im allgemeinen ein Volumenverhältnis von etwa 1 : 100 bis 100 : 1 gewählt wird. Die angewendeten Reaktionstemperaturen liegen in einem Temperaturbereich von 150 bis 380°C, vorzugsweise bei 180 bis 330°C. Feste Ausgangsstoffe der Formel II bzw. III können in Lösungsmitteln gelöst der Reaktion zugeführt werden.

Geeignete Lösungsmittel sind: Methanol, Ethanol, n-Butanol, Tetrahydrofuran, Dioxan, Cyclohexylmethylether, N-Methyl-morpholin, Anisol oder Toluol.

Ein weiterer wirtschaftlicher Vorteil des neuen Verfahrens liegt darin, daß man das bei der Dehydrierung ungesättigter cyclischer Ketone, wie dem Trimethyl-2-cyclohexen-1-on, in technischem Maßstab immer als Nebenprodukt gebildete gesättigte Keton problemlos in den Prozeß zurückführen kann. Mit den bisher verwendeten Katalysatoren war eine Rückführung des entsprechenden Cyclohexanons nur in beschränktem Maße möglich. Mit den neuen Katalysatoren kann man dagegen Trimethylcyclohexanon mit hoher Selektivität zu Trimethylphenol dehydrieren (vgl. Beispiel 1C).

Durch Verwendung eines Palladium- oder Platin-Katalysators auf einem Aluminiumspinell ist es gelungen, die Gasphasendehydrierung von substituierten sechsgliedrigen cyclischen Alkoholen oder Ketonen zu den entsprechenden Phenolen in einem äußerst wirtschaftlich arbeitenden kontinuierlichen Verfahren durchzuführen. Mit dem neuen Verfahren erzielt man hohe Ausbeuten und Selektivitäten an substituierten Phenolen bei außergewöhnlich langen Katalysatorstandzeiten ohne nennenswerten Aktivitätsabfall.

Die nach dem Verfahren der Erfindung hergestellten substituierten Phenole stellen wertvolle Zwischenprodukte für vielfältige Verwendungen dar, speziell 2,3,6-Trimethyl-phenol stellt ein wertvolles Zwischenprodukt für Vitamin E dar (siehe z. B. USP-3 692 839 und GB-1 258 963).

**Beispiel 1**

A. Herstellung des Katalysators

8578 g Aluminiumhydroxid mit einem Gehalt von 77,5 % $Al_2O_3$ und 1776 g technisches Magnesiumoxid mit einem Gehalt von 91,6 % MgO wurden in einem Kneter unter Zugabe von Wasser geknetet bis eine formfähige Masse entstand, was etwa 3 Stunden (h) dauerte. Diese Masse wurde zu 4 mm Strangpreßlingen verformt, 16 h bei 120°C getrocknet und dann 3 h bei 520°C getempert. Das bei 520°C getemperte Material wurde anschließend 8 h lang bei einer Temperatur von 1150°C nachbehandelt. Die Strangpreßlinge bestanden nach dieser Behandlung vorwiegend aus mäßig gut bis gut kristallisiertem Magnesium-Aluminiumspinell, der noch wenig MgO gut kristallin und etwas α-$Al_2O_3$ enthielt. Sie wiesen eine BET-Oberfläche von 17 bis 19,5 $m^2/g$ auf.

1714 g des so gewonnenen Trägermaterials wurden anschliessend in einer Imprägniertrommel mit 497 ml einer Palladiumnitratlösung, die 10,5 g PdO enthielt, imprägniert, das erhaltene Produkt bei 120°C getrocknet und anschließend 3 h auf 300°C erhitzt. Der fertige Katalysator wies eine Oberfläche nach BET von 14 bis 16 $m^2/g$ auf und enthielt 0,5 Gew. % Palladium.

B. Dehydrierung von 2,3,6-Trimethyl-2-cyclohexen-1-on zu 2,3,6-Trimethyl-phenol

In einem auf 280°C erhitzten Reaktor wurden 500 g des gemäß Beispiel 1A hergestellten Katalysators 24 Stunden lang in einem Wasserstoffstrom von 20 Nl pro Stunde aktiviert. Anschließend wurden - ebenfalls bei 280°C-180 g 96 %-iges 2,3,6-Trimethyl-2-cyclohexen-1-on pro Stunde dampfförmig im Gleichstrom mit 20 Nl Wasserstoffstrom pro Stunde über den Katalysator gefahren. Danach wurde das Reaktionsprodukt abgekühlt und gesammelt. Nach 120 Betriebsstunden ergab sich folgende Bilanz:

Es wurden 21,600 kg 96 %-iges 2,3,6-Trimethyl-2-cyclohexen-1-on umgesetzt und 21,478 kg Reaktionsprodukt erhalten, welches zu 85,4 Gew.% (entsprechend 18,342 kg) aus 2,3,6-Trimethyl-phenol und 11,6 Gew.% (entsprechend 2,491 kg) aus 2,3,6-Trimethyl-cyclohexan-1-on bestand. Da das 2,3,6-Trimethyl-cyclohexan-1-on leicht abgetrennt und wieder der Reaktionszone zugeführt oder separat zum 2,3,6-Trimethyl-phenol dehydriert werden kann, erhält man von den gefundenen Daten ausgehend 2,3,6-Trimethyl-phenol in einer Selektivität von 97,5 %.

C. Dehydrierung von 2,3,6-Trimethyl-cyclohexan-1-on zu 2,3,6-Trimethyl-phenol

Über einen gemäß Beispiel 1A hergestellten und gemäß 1B aktivierten Katalysator wurden 180 g eines Gemisches bestehend aus 62 % Trimethylcyclohexanon, 19,5 % Trimethylphenol sowie 18,5 % Leichtsieder (überwiegend Cyclohexanole und geradkettige Ketone) bei 280°C dampfförmig im Gleichstrom mit 20 Nl Wasserstoff pro Stunde geleitet. In 192 Betriebsstunden wurden 34,560 kg Gemisch umgesetzt. Das entspricht den folgenden Mengen an Einsatzstoffen:

| | | |
|---|---|---|
| Trimethylcyclohexanon | 21427,2 g = | 150,9 mol |
| Trimethylphenol | 6739,2 g = | 49,6 mol |
| Leichtsieder | 6393.6 g | |

Es wurden 34,320 kg Reaktionsaustrag mit der folgenden Zusammensetzung erhalten:

| | | |
|---|---|---|
| Trimethylcyclohexanon | 4839,1 g = | 34,1 mol |
| Trimethylphenol | 22136,4 g = | 162,8 mol |
| Leichtsieder | 7344,5 g | |

Der Umsatz von Trimethylcyclohexanon betrug demnach 77,4 % der Theorie; die Selektivität bezüglich Trimethylphenol lag bei 96,9 %.

**Beispiel 2**

Dehydrierung von 2,6-Dimethyl-cyclohexanol zu 2,6-Dimethyl-phenol

In einem Rohrreaktor mit einem Fassungsvermögen von einem Liter wurde ein strangförmiger Katalysator angeordnet, der 0,5 Gew.% Palladiom auf einem Magnesiumaluminiumspinell-Träger enthielt und auf 270°C gebracht (innere Oberfläche nach BET: 122 $m^2/g$). Über dieses Katalysatorbett wurden stündlich 100 g 2,6-Dimethyl-cyclohexanol gasförmig und im Gleichstrom mit 50 Nl Wasserstoff geleitet. Das Reaktionsprodukt wurde - sobald es den Reaktor verließ - abgekühlt und destillativ aufgearbeitet. Man erhielt hierbei aus 100 g 2,6-Dimethyl-cyclohexanol 91,5 g kristallines 2,6-Dimethyl-phenol (Fp. 49°C, Kp. 203°C bei Normaldruck), entsprechend einer Ausbeute von 96 % der Theorie.

Die Umsetzung wurde 60 Tage lang wie oben beschrieben durchgeführt, ohne daß ein Abfall der Katalysatoraktivität festgestellt werden konnte.

**Beispiel 3**

Analog Beispiel 2 wurden an dem dort beschriebenen Katalysator stündlich 100 g einer Mischung, die aus 50 Gew.% 2,6-Dimethyl-cyclohexanol und 50 Gew.% 2,6-Dimethyl-cyclohexanon bestand, bei 250°C unter sonst gleichen Bedingungen umgesetzt. Man erhielt hierbei 2,6-Dimethyl-phenol in einer Ausbeute von 97 % der Theorie.

**Beispiel 4**

Man arbeitete wie in Beispiel 2 beschrieben, jedoch mit einem Katalysator, der 0,5 Gew.% Palladium auf einem Zinkaluminiumspinell-Träger enthielt (BET-Oberfläche 144 $m^2/g$). Ausgehend von 2,6-Dimethyl-3-phenyl-cyclohexanon wurde 2,6-Dimethyl-3-phenylphenol (Kp = 118° C/0,2 mbar) in einer Ausbeute von 94 % der Theorie erhalten.

**Beispiel 5**

Man arbeitete unter den in Beispiel 2 beschriebenen Bedingungen, jedoch mit einem Katalysator, der 0,5 Gew.% Palladium auf einem Cobaltaluminiumspinell-Träger enthielt (BET-oberfläche 138 $m^2/g$). Ausgehend von 2,6-Dimethyl-3-(p-methyl-phenyl)-cyclohexanon wurde 2,6-Dimethyl-3-(p-metyl-phenyl)-phenol (Kp = 129° C/0,2 mbar) in einer Ausbeute von 91 % der Theorie erhalten.

**Beispiel 6**

Man arbeitete unter den in Beispiel 2 beschrieben Bedingungen, verwendet jedoch einen Katalysator, der 0,5 Gew.% Palladium auf einem Nickelaluminiumspinell-Träger enthielt (BET-oberfläche 117 $m^2/g$). Ausgehend von 3-Methyl-2-cyclohexen-1-on wurde m-Kresol (Kp = 202° C) in einer Ausbeute von 97 % der Theorie erhalten.

**Beispiel 7**

In einen Wirbelbettreaktor mit einem Fassungsvermögen von 0,7 Litern wurden 500 ml Katalysator eingefüllt. Der Katalysator enthielt 0,25 Gew.% Platin auf einem Magnesiumaluminiumspinell-Träger und besaß eine Korngröße von 0,2 bis 0,6 mm und eine innere Oberfläche nach BET von 125 $m^2/g$. Die Temperatur des Reaktors wurde auf 275° C eingestellt und eine entsprechend vorerhitzte Mischung aus 200 Litern Stickstoff und 20 Litern Wasserstoff pro Stunde eingeleitet. Durch die so erzeugte Katalysator-Wirbelschicht wurden stündlich und kontinuierlich 60 g 2,6-Dimethyl-cyclohexanol (gasförmig) geführt. Das Reaktionsprodukt wurde durch Kühlung der Abgase gewonnen und destilliert. Aus je 100 g 2,6-Dimethyl-cyclohexanol wurden 90,5 g 2,6-Dimethyl-phenol erhalten, Ausbeute: 95 % der Theorie.

**Beispiel 8**

Man arbeitete wie in Beispiel 7 beschrieben, jedoch bei einer Reaktionstemperatur von 230° C und mit einem Katalysator, der 1,5 Gew.% Palladium auf einem Zinkaluminiumspinell-Träger enthielt. Ausgehend von 2-sec.-Butyl-cyclohexanol wurde 2-sec.-Butyl-phenol (Kp = 227 - 228° C) in einer Ausbeute von 94 % der Theorie erhalten.

**Beispiel 9**

Man arbeitete unter den in Beispiel 7 beschriebenen Bedingungen, verwendete aber einen Katalysator, der 0,5 Gew.% Platin auf einem Magnesiumaluminiumspinell-Träger enthielt. Ausgehend von 2,6-Diisopropylcyclo-hexanol wurde 2,6-Diisopropyl-phenol (Kp = 255 - 256° C) in einer Ausbeute von 96,5 % der Theorie erhalten.

**Patentansprüche**

1. Verbessertes Verfahren zur Herstellung von substituierten Phenolen der allgemeinen Formel I

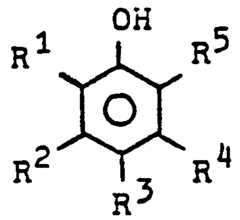

I,

in der $R^1$ bis $R^5$ Wasserstoff oder einen aliphatischen oder aromatischen Kohlenwasserstoffrest bedeuten, durch Dehydrieren eines substituierten cyclischen Alkohols der allgemeinen Formel II

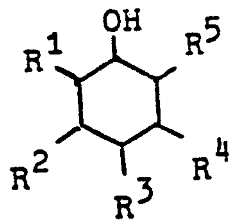

II,

oder eines substituierten cyclischen Ketons der allgemeinen Formel III,

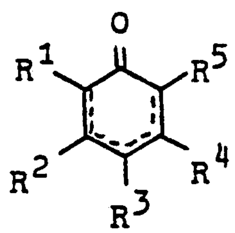

III,

in der eine der gestrichelten Linien für eine zusätzliche C - C - Bindung stehen kann, in der Gasphase bei Temperaturen von 150 bis 380°C an einem Edelmetall-Trägerkatalysator, dadurch gekennzeichnet, daß man als Dehydrierungskatalysator einen Palladium- oder Platinkatalysator auf einem Aluminiumspinell als Katalysatorträger verwendet.

2. Verfahren zur Herstellung von substituierten Phenolen der allgemeinen Formel I gemäß Anspruch 1, dadurch gekennzeichnet, daß man einen Palladium- oder Platinkatalysator auf einem Aluminiumspinell als Katalysatorträger verwendet, der eine innere Oberfläche nach BET von 5 bis 150 aufweist.

3. Verfahren zur Herstellung von substituierten Phenolen der allgemeinen Formel I gemäß Anspruch 1 dadurch gekennzeichnet, daß man für die Dehydrierung von untesättigten cyclischen Ketonen der allgemeinen Formel III einen Palladium- oder Platinkatalysator auf einem Aluminiumspinell als Katalysatorträger verwendet, der eine innere Oberfläche nach BET von 10 bis 50 $m^2/g$ aufweist.

4. Verfahren zur Herstellung von substituierten Phenolen der allgemeinen Formel I gemäß Aspruch 1, dadurch gekennzeichnet, daß man einen Palladium- oder Platinkatalysator auf dem Aluminiumspinell als Katalysatorträger verwendet, der außer Aluminium Magnesium, Zink, Cobalt oder Nickel enthält.

## Claims

1. An improved process for the preparation of a substituted phenol of the formula I

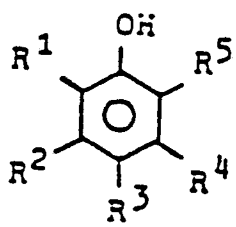

I,

where $R^1$, $R^2$ , $R^3$, $R^4$ and $R^5$ are each hydrogen or an aliphatic or aromatic hydrocarbon radical, by dehydrogenating a substituted cyclic alcohol of the formula II

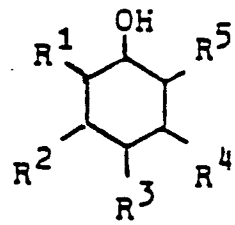

II,

or a substituted cyclic ketone of the formula III

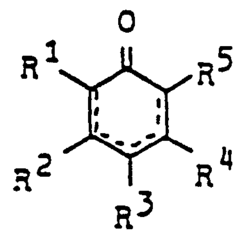

III,

where one of the broken lines can be an additional C - C bond, in the gas phase at from 150 to 380°C over a supported noble metal catalyst, wherein the dehydrogenation catalyst used is palladium or platinum on an aluminum spinel carrier.

2. A process for the preparation of a substituted phenol of the formula I as claimed in claim 1, wherein the catalyst used is palladium or platinum on an aluminum spinel carrier which has a BET specific surface area of from 5 to 150 $m^2/g$.

3. A process for the preparation of a substituted phenol of the formula I as claimed in claim 1, wherein the dehydrogenation of an unsaturated cyclic ketone of the formula III is carried out using a palladium or platinum catalyst on an aluminum spinel carrier which has a BET specific surface area of from 10 to 50 $m^2/g$.

4. A process for the preparation of a substituted phenol of the formula I as claimed in claim 1, wherein the catalyst used is palladium or platinum on an aluminum spinel carrier which, in addition to aluminum, contains magnesium, zinc, cobalt or nickel.

**Revendications**

1. Procédé amélioré pour la préparation de phénols substitués de formule générale

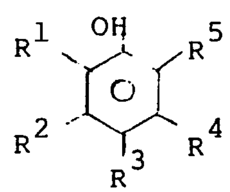

I,

dans laquelle $R^1$ à $R^5$ représentent l'hydrogène ou un reste d'hydrocarbure aliphatique ou aromatique, par déshydrogénation en phase gazeuse, à des températures de 150 à 380°C, sur un catalyseur sur support, en métal noble, d'un alcool cyclique substitué de formule générale II

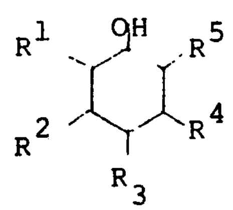

II

ou d'une cetone cyclique substituée de formule générale III

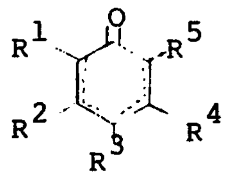

III,

dans laquelle une des lignes en trait interrompu peut représenter une liaison C - C additionnelle, caractérisé par le fait que l'on utilise, comme catalyseur de déshydrogénation, un catalyseur de palladium ou platine sur un spinelle d'aluminium comme support de catalyseur.

2. Procédé pour la préparation de phénols substitués de formule générale I selon la revendication 1, caractérisé par le fait que l'on utilise un catalyseur de palladium ou platine sur un spinelle d'aluminium comme support de catalyseur qui présente une surface intérieure selon BET de 5 à 150 $m^2/g$.

3. Procédé pour la préparation de phénols substitués de formule générale I selon la revendication 1, caractérisé par le fait que, pour la déshydrogénation de cétones cycliques insaturés de formule générale III, on utilise un catalyseur de palladium ou platine sur un spinelle d'aluminium comme support de catalyseur qui présente une surface intérieure selon BET de 10 à 50 $m^2/g$.

4. Procédé pour la préparation de phénols substitués de formule générale I selon la revendication 1, caractérisé par le fait que l'on utilise un catalyseur de palladium ou platine sur un spinelle d'aluminium comme support de catalyseur, qui contient, outre l'aluminium, du magnésium, zinc, cobalt ou nickel.